# EUROPÄISCHE PATENTANMELDUNG

(11) **EP 0 893 112 A1**
(43) Veröffentlichungstag der Anmeldung: **27.01.1999**
(21) Anmeldenummer: 98113689.8
(22) Anmeldetag: 22.07.1998
(51) Int. Cl.: A61F 5/14, B29C 45/16, A43B 7/14

(54) **Orthopädisches Stützteil**

(30) Priorität: 23.07.1997 AT 459/97 U
(71) Anmelder: Globus K. Kremendahl GmbH & Co. KG, 42897 Remscheid (DE)
(72) Erfinder: Kremendahl, Jürgen, Remscheid (DE); Pliessnig, Gerhard, Fulpmes (AT)
(74) Vertreter: Bardehle, Heinz, Dipl.-Ing.

(57) **Zusammenfassung**

Es wird ein Verfahren zur Herstellung eines orthopädischen Stützteils, insbesondere einer orthopädischen Schuheinlage beschrieben. Das Verfahren weist zumindest zwei miteinander verbundene Schichten aus zumindest teilweise aus Kunststoff bestehenden Materialien auf, wobei mittels eines Spritzgießverfahrens auf eine erste Schicht eine zweite Schicht aufgespritzt wird, wobei die zweite Schicht in einem Mehrkomponenten-Spritzgießverfahren ohne Umsetzen des Spritzteils auf die erste Schicht aufgespritzt wird.

## Beschreibung

Die Erfindung betrifft ein orthopädisches Stützteil, insbesondere für eine orthopädische Schuheinlage, welches zumindest zwei miteinander verbundene Schichten aus zumindest teilweise aus Kunststoff bestehenden Materialien aufweist, und ein Verfahren zur Herstellung des orthopädischen Stützteils.

Bei herkömmlichen derartigen orthopädischen Stützteilen sind die Schichten miteinander verklebt. Dies führt zwar im allgemeinen zu befriedigenden Ergebnissen. Aufgabe der Erfindung ist es jedoch, die Herstellung solcher Stützteile zu vereinfachen und die Haftung zwischen den Schichten des Stützteils zu verbessern.

Erfindungsgemäß wird dies dadurch erreicht, daß die zweite Schicht auf die erste Schicht aufgespritzt ist.

Beim Aufspritzen wird das Material der zweiten Schicht in flüssiger Form auf die erste Schicht aufgebracht und härtet auf dieser aus, wobei es eine innige Verbindung mit dem Material der ersten Schicht eingehen kann.

Vorteilhaft kann noch auf die zweite Schicht während desselben Arbeitsganges eine Deckschicht aus Leder, Textil, Vlies oder einem weichen Kunststoff aufgebracht werden. Dies kann dadurch geschehen, daß diese Deckschicht vor dem Schließen der Form zwischen die beiden Formplatten gespannt und dann beim Spritzvorgang innig mit dem Elastomer verbunden wird.

Besonders vorteilhaft ist es, wenn als Spritzgießverfahren zur Herstellung des Stützteils ein sogenanntes Zwei- oder Mehrkomponenten-Spritzgießverfahren verwendet wird, wobei die einzelnen Verfahrensschritte automatisch durchgeführt werden können. Besonders geeignet ist das sogenannte Drehtischverfahren. Bei diesem wird zunächst die erste Schicht hergestellt, indem ein geeignetes Kunststoffmaterial in die Form eingespritzt wird. Im folgenden wird die Form geöffnet, wobei das Produkt in der einen Formhälfte verbleibt. Diese Formhälfte wird verschwenkt und eine andere Gegenformhälfte, welche die entsprechenden Ausnehmungen für die zweite Schicht aufweist, wird an die Formhälfte mit der ersten Schicht angelegt. Die zweite Schicht kann nun an die erste Schicht angespritzt werden. Spritzgießmaschinen für die Ausführung solcher Mehrkomponenten-Spritzgießverfahren sind kommerziell erhältlich.

Weitere Vorteile und Einzelheiten der Erfindung werden im folgenden anhand den beiliegenden Zeichnungen erläutert, in welchen
- **Figur 1**: ein orthopädisches Stützteil gemäß einer bevorzugten Ausführungsform der Erfindung zeigt, und
- **Figur 2**: ein orthopädisches Stützteil für einen Kinderfuß gemäß der Erfindung zeigt.

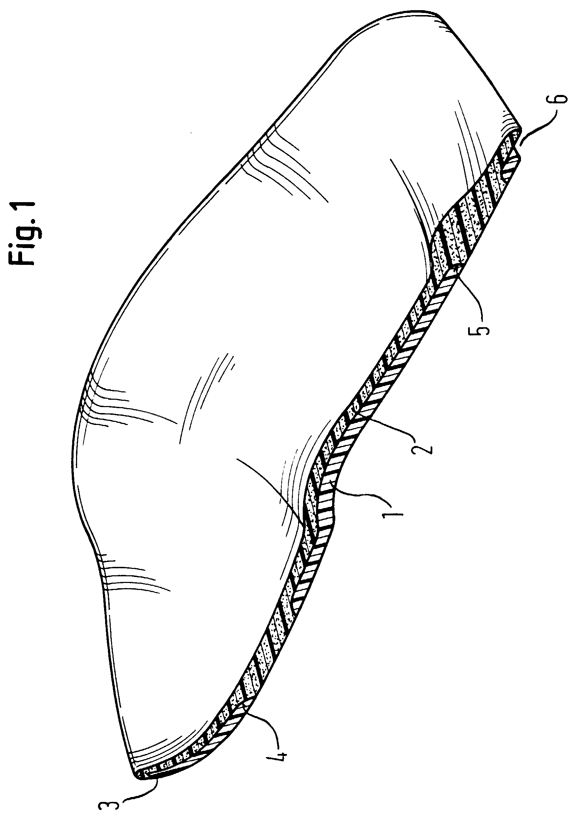

Figur 1 zeigt eine dreidimensionale Darstellung eines Teils einer erfindungsgemäßen Schuheinlage, welche in Längsrichtung entlang ihrer Mittelebene geschnitten ist.

Die erste Schicht 1, die vom abzustützenden Fuß abgewandt angeordnet ist, besteht aus einem härteren Material als die zweite Schicht 2, beispielsweise einem formstabilen Thermoplast oder Elastomer. Die erste Schicht 1 übernimmt somit die Stützfunktion und die zweite Schicht 2 eine Dämpfungsfunktion.

Im in der Figur 1 gezeigten Ausführungsbeispiel ist der Rand 3 der ersten Schicht 1 im rückwärtigen und seitlichen Bereich der Schuheinlage von der zweiten Schicht 2 umbördelt, wodurch der Tragekomfort verbessert wird.

Im Fersenbereich ist eine Aussparung 4 in der ersten Schicht 1 vorgesehen. Die Unterseite der Schuheinlage wird im Bereich der Aussparung 4 von der zweiten Schicht 2 gebildet. Dadurch wird die Dämpfung im Fersenbereich erhöht. Außerdem weist Schicht 2 einen hohen Reibungskoeffizienten auf, wodurch ein Verrutschen der Schuheinlage während des Tragens verhindert wird.

Eine zweite Aussparung 5 in der Schuheinlage in ihrem vorderen Bereich kann vorgesehen sein. Auch hier wird die Unterseite der Schuheinlage von der zweiten Schicht 2 gebildet, wodurch eine starke Dämpfung im Metatarsalbereich des Fußes erreicht wird. Die Schicht 2 kann im Bereich der Aussparung 5 nach ihrer dem Fuß zugewandten Seite eine pelottenförmige Verdickung aufweisen, wodurch das Mittelfußgewölbe des Fußes (Metatarsalbereich) abgestützt wird.

Diese pelottenförmige Verdickung kann auch dann Bestandteil der Einlage sein, wenn die Schicht 1 nach unten nicht ausgespart ist. Nach oben kann in jedem Fall eine pelottenförmige Verdickung vorgesehen werden.

Man kann folglich ein Pelottenloch unten in den harten Kern einarbeiten, und dann druckt sich die Schicht 2 nach unten durch. Nach oben kann sie in Form einer Pelotte eine Verdickung aufweisen.

Zur Ausbildung eines Übergangsbereiches 6 ragt die zweite Schicht 2 an der Vorderseite der Schuheinlage über den Rand der ersten Schicht 1 vor (maximal bis zur Gesamtlänge einer Schuh-Einlegesohle).

Zur Ausbildung eines unterschiedlich steifen Bereichs eines erfindungsgemäßen Stützteils kann die Dicke der ersten Schicht 1 über das Stützteil variieren. Ebenso sind Variationen in der Dicke der zweiten Schicht 2 möglich, um die Dämpfungseigenschaften an die jeweiligen Bedürfnisse anzupassen. Beispielsweise kann die zweite Schicht 2 im Fersenbereich einer Schuheinlage eine Verdickung aufweisen.

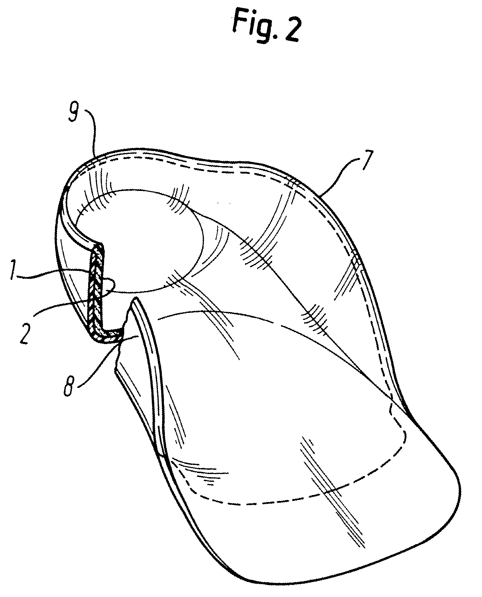

Eine weitere Variante der Erfindung ist in Figur 2 gezeigt, in der ein orthopädisches Stützteil dargestellt ist, insbesondere für einen Kinderfuß, mit hohem Längsgewölbe, hinterem Fersenrand und hochgezogenem Innenrand 7 und Außenrand 8.

Der Aufbau in zwei Schichten erfolgt wie bei der zuvor beschriebenen Schuheinlage.

Diese Einlage ist zur Behandlung des kindlichen Knickfußes bzw. Knick-Senkfußes vorgesehen.

Der hochgezogene Innenrand 7 mit entsprechender anatomischer Formgebung im Längsgewölbe dient der Abstützung des nach innen abknickenden Fersenbeins.

Damit die Ferse nach der Aufrichtung nicht nach außen wegrutschen kann, wirkt der hochgezogene Außenrand 8 als Gegenhalt.

Ein hinterer Fersenrand 9 bewirkt eine zusätzliche Stabilisierung des Rückfußes.

Des weiteren ist der stützende Unterbau im Fersenbereich so gestaltet, daß eine flächige Auflage ein Abkippen der Einlage nach innen verhindert.

Die Merkmale der beschriebenen Ausführungsformen können auch miteinander kombiniert werden.

## Patentansprüche

1. Verfahren zur Herstellung eines orthopädischen Stützteils, insbesondere einer orthopädischen Schuheinlage, welches zumindest zwei miteinander verbundene Schichten aus zumindest teilweise aus Kunststoff bestehenden Materialien aufweist, **dadurch gekennzeichnet**, daß mittels eines Spritzgießverfahrens auf eine erste Schicht (1) eine zweite Schicht (2) aufgespritzt wird, wobei die zweite Schicht (2) in einem Mehrkomponenten-Spritzgießverfahren ohne Umsetzen des Spritzteils auf die erste Schicht (1) aufgespritzt wird.

2. Verfahren nach Anspruch 1, dadurch gekennzeichnet, daß die erste Schicht (1) ebenfalls im Spritzgießverfahren hergestellt ist.

3. Verfahren nach Anspruch 1 oder 2, dadurch gekennzeichnet, daß das Mehrkomponenten-Spritzgießverfahren das Drehtischverfahren ist.

4. Verfahren nach einem der vorangegangen Ansprüche, dadurch gekennzeichnet, daß auf die zweite Schicht (2) eine Deckschicht aus Leder, Textil, Vlies oder einem weichen Kunststoffmaterial aufgebracht wird.

5. Orthopädisches Stützteil, insbesondere orthopädische Schuheinlage, welches zumindest zwei miteinander verbundene Schichten aus zumindest teilweise aus Kunststoff bestehenden Materialien aufweist, **dadurch gekennzeichnet**, daß die zweite Schicht (2) auf die erste Schicht (1) aufgespritzt ist.

6. Orthopädisches Stützteil nach Anspruch 5, dadurch gekennzeichnet, daß die vom abzustützenden Körperteil abgewandte erste Schicht (1) härter als die zweite Schicht ist.

7. Orthopädisches Stützteil nach Anspruch 6, dadurch gekennzeichnet, daß die erste Schicht aus einem formstabilen Thermoplast oder Elastomer besteht.

8. Orthopädisches Stützteil nach Anspruch 6 oder 7, dadurch gekennzeichnet, daß die dem abzustützenden Körperteil zugewandte zweite Schicht (2) aus einem Elastomer besteht.

9. Orthopädisches Stützteil nach einem der Ansprüche 5 bis 8, dadurch gekennzeichnet, daß der Rand (3) der ersten Schicht (1) von der zweiten Schicht (2) teilweise oder vollständig umbördelt ist.

10. Orthopädisches Stützteil nach einem der Ansprüche 5 bis 9, dadurch gekennzeichnet, daß die erste Schicht (1) eine erste Aussparung (4) aufweist, wobei die Unterseite des Stützteils im Bereich der Aussparung (4) von der zweiten Schicht (2) gebildet wird und/oder daß eine zweite Aussparung (5) im Metatarsalbereich vorgesehen ist.

11. Orthopädisches Stützteil nach Anspruch 10, dadurch gekennzeichnet, daß sich die Ausnehmung (4) zur Ausbildung eines Dämpfungsbereiches und/oder zur Vermeidung des Rutschens einer Schuheinlage im Schuh im Fersenbereich der Schuheinlage befindet.

12. Orthopädisches Stützteil nach Anspruch 10 oder 11, dadurch gekennzeichnet, daß die Unterseite der zweiten Schicht (2) im Bereich der Aussparung (4) der ersten Schicht (1) einen hohen Reibungskoeffizienten aufweist.

13. Orthopädisches Stützteil nach einem der Ansprüche 5 bis 12, dadurch gekennzeichnet, daß zur Ausbildung unterschiedlich steifer Bereiche des Stützteils die Dicke der ersten Schicht (1) variiert.

14. Orthopädisches Stützteil nach einem der Ansprüche 5 bis 13, dadurch gekennzeichnet, daß zur Anpassung der Dämpfungseigenschaften des Stützteils die Dicke der zweiten Schicht (2) variiert.

15. Orthopädisches Stützteil nach Anspruch 14, dadurch gekennzeichnet, daß zur Ausbildung eines Dämpfungselementes die zweite Schicht (2) im Fersenbereich einer Schuheinlage eine Verdickung aufweist.

16. Orthopädisches Stützteil nach einem der Ansprüche 5 bis 15, dadurch gekennzeichnet, daß die zweite Schicht (2) in einem Bereich (6) über den Rand der ersten Schicht (1) hinausragt.
